# EUROPÄISCHE PATENTSCHRIFT

(11) **EP 2 094 105 B1**
(45) Veröffentlichungstag und Bekanntmachung des Hinweises auf die Patenterteilung: **26.10.2011**
(21) Anmeldenummer: 07856636.1
(22) Anmeldetag: 12.12.2007
(51) Int. Cl.: A23L 1/00, A23L 1/22

(54) **ESSBARE FOLIENFÖRMIGE ZUBEREITUNG MIT COLA-GESCHMACK**
EDIBLE FOIL-SHAPED COKE-FLAVORED PREPARATION
PRÉPARATION PELLICULAIRE COMESTIBLE, AU GOÛT DE COCA

(30) Priorität: 22.12.2006 DE 102006061287
(43) Veröffentlichungstag der Anmeldung: 02.09.2009
(73) Patentinhaber: LTS LOHMANN Therapie-Systeme AG, 56626 Andernach (DE)
(72) Erfinder: HOFFMANN, Hans-Rainer, 56566 Neuwied (DE); ASMUSSEN, Bodo, 56170 Bendorf-Sayn (DE); PIOTROWSKI, Holger, 56575 Weissenthurm (DE); MÜLLER, Markus, 53840 Troisdorf (DE)
(86) Internationale Anmeldenummer: PCT/EP2007/010888
(87) Internationale Veröffentlichungsnummer: WO 2008/077488

(56) Entgegenhaltungen:
- EP-A- 1 589 067
- WO-A-98/26764
- WO-A-2004/019698
- WO-A-2004/057985
- WO-A-2006/086560
- FR-A- 1 580 138
- US-A1- 2004 202 698
- US-A1- 2004 265 359

## Beschreibung

Die Erfindung betrifft eßbare folienförmige Zubereitungen mit Cola-Geschmack, die bei Kontakt mit Feuchtigkeit schnell und rückstandsfrei zerfallen.

Die vorliegende Erfindung betrifft den Bereich der Süßwaren und Knabberartikel für den menschlichen Konsum. Üblicherweise werden Süßwaren in Form von Riegeln, Bonbons, Kaubonbons, Kaugummis, Chips, Keksen und dergleichen in einer Vielzahl von Geschmacksrichtungen angeboten. Die meisten dieser Produkte müssen gekaut oder gelutscht werden, um beim Konsumenten das gewünschte Geschmackserlebnis zu erzeugen. Daher ist ihr Konsum vergleichsweise auffällig und gilt bei vielen Gelegenheiten als unpassend, z. B. im Unterricht oder bei Besprechungen. Als weitere Nachteile der konventionellen Süßwaren werden angesehen, daß sie in Hosen-, Hemden- oder Jackentaschen auftragen, zu unerwünschten Verschmutzungen führen können und ein Gefühl von Stückchen oder Krümeln im Mund und/oder an den Zähnen hinterlassen.

Der vorliegenden Erfindung lag daher die Aufgabe zugrunde, ein Produkt bereitzustellen, das die vorstehend genannten Nachteile nicht aufweist, zu einem erfrischenden Geschmackserlebnis führt, welches dem eines Schlucks Cola nahekommt, und dabei ein angenehmes Mundgefühl bietet.

Diese Aufgabe wird durch die Bereitstellung einer eßbaren, folienförmigen, bei Kontakt mit Feuchtigkeit schnell und rückstandsfrei zerfallenden Zubereitung (nachfolgend als "Wafer" bezeichnet) mit Cola-Geschmack gelöst, welche hydroxypropylierte Tapiokastärke oder hydroxypropylierte Tapiokastärke in Kombination mit weiteren filmbildenden Polymeren enthält.

Hauchdünne Streifen, die ohne Lutschen oder Kauen ein kühles, atemfrisches Geschmackserlebnis auf der Zunge hinterlassen, sind bekannt. Sie werden beispielsweise in den Geschmacksrichtungen "Peppermint", "Wild-Mint" und "Lemon-Frost" von der Firma Wrigley unter dem Namen ECLIPSE FLASH^{™} oder von der Firma Pfizer in den Geschmacksrichtungen "Cool Mint®", "FreshBurst®", Cinnamon und "Fresh Citrus" unter dem Markennamen Listerine PocketPaks® im Handel angeboten.

Die unter der Veröffentlichungsnummer US 2003/0224090 A1 offengelegte Patentanmeldung beschreibt Knabberartikel in Form von im Mund löslichen, eßbaren, Folien, die eine oder mehrere Lagen umfassen und im Mundraum schnell und rückstandsfrei auflösbar sein sollen. Obwohl in dieser Patentanmeldung viele natürliche und künstliche Geschmacksstoffe genannt werden, die in den beschriebenen eßbaren Folien auf Basis von filmbildenden Polymeren enthalten sein können, um ein Geschmackserlebnis beim Konsumenten hervorzurufen, finden sich keine Hinweise auf Cola-Aromen in diesem Dokument.

Um eine sich im Mund bei Kontakt mit Feuchtigkeit, insbesondere bei Kontakt mit Speichel, schnell auflösende und zu einem erfrischenden Cola-Geschmackserlebnis führende folienförmige Zubereitung bereitstellen zu können, wurde das bei der Herstellung von in wäßrigen Medien schnell und rückstandsfrei löslichen Folien verwendbare Prozeßlösemittel Wasser in einer ersten Versuchreihe durch über den Einzelhandel als Erfrischungsgetränk erhältliche Cola ersetzt (siehe Beispiel 1).

Bei diesen Versuchen stellte sich jedoch heraus, daß die aus diesem Verfahren resultierenden Wafer keinen oder lediglich einen kaum wahrnehmbaren Cola-Geschmack aufweisen. Möglicherweise war der Cola-Anteil in dem Wafer zu niedrig, um zu einem zufriedenstellenden Geschmackserlebnis führen zu können. Allerdings läßt sich der Anteil am Prozeßlösemittel nicht beliebig erhöhen, weil die lösemittel- und polymerhaltige Masse eine Mindestviskosität aufweisen muß, bevor sie für die Weiterverarbeitung zu eßbaren Folien auf einer Unterlage ausgestrichen werden kann.

Ein Aufkonzentrieren der Aromastoffe der Erfrischungsgetränke durch Entfernen von Wasser (z. B. durch Abdestillieren) führt zu keinem zufriedenstellenden Ergebnis; durch den hohen Zuckeranteil im Erfrischungsgetränk - und somit auch im daraus gewonnenen Konzentrat - kann es bei der für ein ansprechendes Geschmackserlebnis einzusetzenden Menge zu Unverträglichkeiten in der Folie kommen. Beispielsweise kann der Zucker kristallisieren. Versuche mit,zuckerfreien "light"-Varianten der Erfrischungsgetränke zeigten unverkennbar, daß mit dem Wasser auch wesentliche Aromakomponenten entfernt werden, was wiederum das Geschmackserlebnis beeinträchtigt.

In Erwartung, daß sich der Verlust an Aromastoffen beim Aufkonzentrieren einer zuckerfreien oder zuckerreduzierten Version des Cola-Getränks umgehen lassen sollte, wurde in einer weiteren Versuchsreihe ein handelsübliches Cola-Konzentrat verwendet, bei dem es sich um einen Sirup handelt, der zur Zubereitung eines Erfrischungsgetränks mit einer vorgegebenen Menge Wasser verdünnt werden soll. Gemäß Gebrauchsanweisung lassen sich mit 500 ml des Cola-Sirups 18 Liter Getränk herstellen. Wafer, die unter Verwendung dieses Sirups hergestellt worden waren, wiesen zwar einen deutlichen Cola-Geschmack auf, hatten aber aufgrund der immer noch hohen Zuckerkonzentration auch einen unangenehm süßen Geschmack (siehe Beispiel 2).

Auch bei Verwendung eines ebenfalls kommerziell erhältlichen Cola-Sirups, bei dem ein Teil des Zuckers gegen Süßstoff ausgetauscht war, und eines "light"-Sirups, der nur Süßstoffe enthielt, aber keinen Zucker, war der Anteil an Süßstoff in den Wafern so hoch, daß sich der für die in den Sirupen enthaltenen Süßstoffe typische Nachgeschmack unangenehm bemerkbar machte.

Als weiterer Nachteil hat sich herausgestellt, daß die mit Cola-Sirup hergestellten eßbaren Folien eine unerwünschte Klebrigkeit und Weichheit aufwiesen, die sie als Produkt für den Süßwarenbereich ungeeignet erschienen ließen.

Aufgrund dieser Versuchsergebnisse wurde überraschenderweise herausgefunden, daß sich eßbare Folien, die einen zufriedenstellenden Cola-Geschmack aufweisen, wesentlich bessere haptische Eigenschaften haben und sich schnell und rückstandsfrei im Mund auflösen, durch die Verwendung von Cola-Aromen, wie sie in der Lebensmittelindustrie für die Aromatisierung von Eis, Backwaren oder Mixgetränken verwendet werden, herstellen lassen.

Die erfindungsgemäßen Wafer umfassen als essentielle Bestandteile mindestens ein Cola-Aroma sowie mindestens ein filmbildendes Polymer, nämlich hydroxypropylierte Tapiokastärke oder hydroxypropylierte Tapiokastärke in Kombination mit weiteren filmbildenden Polymeren.

Für die gewünschten physikalischen Eigenschaften der folienförmigen Zubereitung, beispielsweise deren Flexibilität, sind zudem mindestens eine emulgierende Komponente und/oder eine weichmachende Komponente notwendig. Allerdings können bestimmte Inhaltsstoffe für eine folienförmige Zubereitung mehrere Eigenschaften haben, auch wenn diese polyfunktionellen Verbindungen eigentlich für einen anderen Zweck eingesetzt werden. So hat beispielsweise das filmbildende Polymer Polyvinylalkohol (PVA) auch emulgierende Eigenschaften, oder der Süßstoff Zucker kann in Formulierungen zusätzlich als Weichmacher fungieren. Daher können auch folienförmige Zubereitungen verwirklicht werden, die keinen separaten Emulgator oder Weichmacher enthalten. Somit sind separate Emulgatoren und Weichmacher nicht als essentielle Komponenten der erfindungsgemäßen filmförmigen Zubereitungen anzusehen. In bevorzugten Ausführungsformen enthalten die Wafer jedoch zusätzlich zu dem filmbildenden Polymer oder Polymergemisch und dem Cola-Aroma mindestens einen Emulgator, mindestens einen Weichmacher, mindestens ein Säuerungsmittel, einen oder mehrere Süßstoffe, mindestens einen Farbstoff, einen oder mehrere weitere Aromastoffe, einen oder mehrere Füllstoffe und/oder mindestens ein Konservierungsmittel.

Die Ansprüche, die an das filmbildende Polymer oder an die filmbildenden Polymere für die folienförmigen Zubereitungen gestellt werden, ergeben sich aus der Aufgabenstellung. Die filmbildenden Polymere, die Grundlage für die erfindungsgemäßen folienförmigen Zubereitungen sind, müssen sich schnell und rückstandsfrei im Mund auflösen. Darüber hinaus müssen die filmbildenden Polymere kompatibel mit den jeweils verwendeten Aromen sein, d. h. das filmbildende Polymer oder die Kombination filmbildender Polymere muß die Aroma- bzw. Geschmacksstoffe in ausreichendem Maße aufnehmen und stabilisieren können. Weiterhin darf das Geschmackserlebnis nicht durch die filmbildenden Polymere negativ beeinflußt werden. Die filmbildenden Polymere dürfen daher weder einen wahrnehmbaren Eigengeschmack haben, noch dürfen sie das durch das zugefügte Aroma hervorzurufende Geschmackserlebnis in anderer Weise beeinträchtigen.

Aufgrund des gewünschten schnellen Auflöseverhaltens der erfindungsgemäßen Zubereitung ist die Auswahl der für ihre Herstellung in Frage kommenden Polymere auf Cellulosederivate (z.B. Natriumcarboxymethylcellulose, Hydroxypropylmethylcellulose, Hydroxypropylcellulose), teilhydrolysierte Polyvinylalkohole, Polyvinylpyrrolidon, Gelatine, Alginate, Polyethylenglykole sowie wasserlösliche Stärkeanteile und wasserlösliche Stärkederivate wie hydroxypropylierte Stärke, z.B. hydroxypropylierte Erbsenstärke, beschränkt.

Die im Rahmen der vorliegenden Erfindung durchgeführten Untersuchungen zeigten jedoch, daß Folien auf Basis von Polyvinylpyrrolidon klebrig und weich, Folien auf Basis von teilhydrolysiertem Polyvinylalkohol anfällig für Feuchtigkeit (hygroskopisch) und weich sind. Zudem lösen sich diese Folien zu langsam auf. Die wasserlöslichen Cellulosederivate führten zwar zu Folien mit zufriedenstellenden physikalischen Eigenschaften, hatten jedoch negative Einflüsse auf das in diesen Folien enthaltene Cola-Aroma. Der Eigengeschmack dieser Cellulosederivate wurde als störend empfunden.

Überraschenderweise wurde gefunden, daß sich mit hydroxypropylierter Tapiokastärke Cola-Wafer mit den gewünschten Eigenschaften herstellen lassen.

Tapiokastärke, auch Maniokstärke genannt, wird aus bearbeiteten und getrockneten Wurzeln der Maniokpflanze (*Manihot utilissima* und *Manihot palmata*) gewonnen. Tapiokastärke weist üblicherweise einen Amylosegehalt von etwa 17 Gew.-% und einen Amylopektingehalt von etwa 83 Gew.-% auf, jeweils bezogen auf das Trockengewicht der Stärke. Teilhydrolysierte Tapiokastärke wird in der Lebensmittelindustrie als Zusatzstoff (E 1440) verwendet.

Mit hydroxypropylierter Tapiokastärke läßt sich mit den meisten Aroma-Mischungen (Cola-Aroma, Cola-Sirup) eine verarbeitbare Masse herstellen, die zu stabilen Folien weiterverarbeitet werden kann. Diese Folien lösen sich im Mund in entsprechend kurzer Zeit rückstandsfrei auf und beeinträchtigen auch das Cola-Geschmackserlebnis nicht.

Zusätzlich zur Tapiokastärke können weitere filmbildende Polymere verwendet werden, um verarbeitbare Massen herzustellen, die zu Wafern mit gewünschten Eigenschaften weiterverarbeitet werden können. Als zusätzliche filmbildende Polymere können vorzugsweise die bereits genannten wasserlöslichen Cellulosederivate, teilhydrolysierte Polyvinylalkohole, Polyvinylpyrrolidon, Gelatine, Alginate, Polyethylenglykole sowie wasserlösliche Stärkeanteile und andere wasserlösliche Stärkederivate verwendet werden.

Vorzugsweise beträgt der Anteil an filmbildenden Polymeren in der filmförmigen Zubereitung 55 bis 75 Gew.-%, bezogen auf die Trockenmasse der folienförmigen Zubereitung. Besonders bevorzugt beträgt der Anteil an hydroxypropylierter Tapiokastärke 55 bis 65 Gew.-% und der Anteil an einem oder mehreren weiteren filmbildenden Polymeren in der Trockenmasse 0,01 bis 10 Gew.-%.

Als Cola-Aroma kommen die in der Lebensmittelindustrie verwendeten Konzentrate in Betracht, die für die Aromatisierung von Eis, Backwerk oder Mixgetränken vorgesehen sind. Durch Verwendung dieser Aromen können die mit einer zu hohen Zuckerfracht einhergehenden Probleme, die bei Cola-Sirupen oder Cola-Konzentraten vorliegen, umgangen werden. Zudem können Farbstoffe und weitere geschmacksgebende Komponenten in gewünschter Dosierung zugemischt werden, z.B. Coffein, um den Wafer zu optimieren. Vorzugsweise ist das Cola-Aroma in einer Menge von 5 bis 20 Gew.-% in der Trockenmasse der folienförmigen Zubereitung enthalten.

In bevorzugten Ausführungsformen enthält der Wafer mindestens einen Emulgator, der das Mundgefühl und den Geschmackseindruck zusätzlich verbessert. Vorzugsweise ist der Emulgator bzw. sind die Emulgatoren aus der Gruppe ausgewählt, die Mono- und Diglyceride von Speisefettsäuren (d. h. ungesättigte Fettsäuren mit bis zu 24 Kohlenstoffatomen sowie einfach und mehrfach ungesättigte Fettsäuren mit bis zu 22 Kohlenstoffatomen), Polyethylenglykolether, Sorbitanfettsäureester, Polysorbate, Pectine und Lecithin umfaßt. Beispiele für Polyalkylenglykolether, sogeanannte Fettalkoholethoxylate und kommerziell auch unter dem Namern Brij^{®} erhältlich, sind Polyoxyethylen(2)stearylether (Brij^{®} 72), Polyoxyethylen(4)laurylether (Brij^{®} 30), Polyoxyethylen(10)stearylether (Brij^{®} 76), Polyoxyethylen(10)-cetylether (Brij^{®} 56), Polyoxyethylen(20)stearylether (Brij^{®} 78) und Polyoxyethylen(23)laurylether (Brij^{®} 35). Beispiele für Sorbitanfesttsäureester, kommerziell auch unter dem Namen Span^{®} erhältlich, sind Sorbitanmonolaurat (Span^{®} 20), Sorbitanmonopalmitat (Span^{®} 40), Sorbitanmonostearat (Span^{®} 60), Sorbitantristearat (Span^{®} 65) und Sorbitanmonooleat (Span^{®} 80). Beispiele für Polysorbate sind Polyoxyethylen-20-sorbitanmonolaurat (Polysorbat 20, Tween^{®} 20), Polyoxyethylen-40-sorbitanmonopalmitat (Polysorbat 40, Tween^{®} 40), Polyoxyethylen-60-sorbitanmonostearat (Polysorbat 60, Tween^{®} 60), Polyoxyethylen-65tristearat (Polysorbat 65, Tween^{®} 65) und Polyoxyethylen-80-sorbitanmonooleat (Polysorbat 80, Tween^{®} 80). Als geeigneter Emulgator kann zudem Macrogolglycerolhydroxystearat (Poly(oxyethylen)-40-hydriertes Rizinusöl) verwendet werden.

Der Anteil an Emulgator(en) in der Trockenmasse der folienförmigen Zubereitung beträgt vorzugsweise zwischen 0 und 7 Gew.-%.

In einer bevorzugten Ausführungsform enthält die erfindungsgemäße folienförmige Zubereitung mindestens einen Weichmacher, der die Flexibilität der eßbaren folienförmigen Zubereitung verbessert.

Die für die eßbaren folienförmigen Zubereitungen geeigneten Weichmacher sind vorzugsweise aus der Gruppe ausgewählt, die Ethylenglycole, Polyethylenglycole, Dibutylsebacat, Diethylphtahalat, diacetylierte Monoglyceride, Triacetin, Tributylcitrat, Triethylcitrat, Acetyltributylcitrat, Acetyltriethylcitrat, Benzoylbenzoat, Propylenglycol, Ricinusöl, Saccharose, Isomalt, Mannitol, Stärkezucker und Dexpanthenol umfaßt.

Besonders bevorzugt werden wasserlösliche Weichmacher eingesetzt, um mit dem filmbildenden Polymer gut mischbar zu sein. Aufgrund der Anforderungen an die Kompatibilität, die Unbedenklichkeit und die Neutralität für das Geschmackserlebnis werden Sorbidex und Glycerin als Weichmacher besonders bevorzugt. Der Gehalt an Weichmacher in der Zubereitung beträgt zwischen 0 und 18 Gew.-%, bezogen auf die Trockenmasse der folienförmigen Zubereitung.

Es wurde herausgefunden, daß der Zusatz eines Säuerungsmittels für den geschmacklichen Gesamteindruck vorteilhaft ist. Durch den sauren Geschmack wird der Speichelfluß angeregt und man erzielt das angestrebte Erfrischungsgefühl eher. Als Säuerungsmittel kommen insbesondere Weinsäure, Ascorbinsäure, Äpfelsäure, Phosphorsäure, Milchsäure und Zitronensäure in Frage, wobei Zitronensäure das besonders bevorzugte Säuerungsmittel ist. Das Säuerungsmittel kann in einer Menge von 0 bis 5 Gew.-% in der Trockenmasse der folienförmigen Zubereitung enthalten sein.

Durch die Auswahl und die Konzentration des Süßstoffs oder der Süßstoffe, die in der folienförmigen Zubereitung enthalten sein können, lassen sich die Eigenschaften und der Geschmack des Wafers an unterschiedliche Erfordernisse und Vorlieben anpassen.

Als Beispiele für Süßstoffe, die dem Grunde nach für die Herstellung der erfindungsgemäßen Wafer geeignet sind, können Acesulfam, Aspartam, Cyclamat, Saccharin, Sorbitol, Sucralose (Trichlorsucrose), Thaumatin, Neohesperidin DC oder Mischungen dieser Süßstoffe genannt werden.

Vorzugsweise wird als Süßstoff Sucralose verwendet, da sie, anders als beispielsweise Saccharin, keinen bitteren Nachgeschmack, sondern einen angenehmen Geschmack hat.

Der Süßstoff bzw. die Süßstoffe ist/sind vorzugsweise in einer Menge von 0 bis 19 Gew.-% in der Trockenmasse der folienförmigen Zubereitung enthalten.

Durch Zusatz von weiteren Aromen, z. B. Coffein, Zitronenaroma, Limetten-Aroma, läßt sich der geschmackliche Gesamteindruck des erfindungsgemäßen Wafers abstimmen. Der Anteil an zusätzlichen Aromen in der Trockenmasse der folienförmigen Zubereitung kann bis zu 3 Gew.-% betragen.

Ferner kann der Zubereitung auch ein Farbstoff zugesetzt werden, um die Wafer beispielsweise entsprechend der charakteristischen Cola-Färbung zu färben. Hierfür eignet sich Zuckerkulör (E 150) besonders gut. Es ist aber auch möglich, den ansonsten farblosen Folien durch Verwendung unterschiedlicher Farbstoffe einen beliebigen Farbton zu geben.

Vorzugsweise ist der Farbstoff Menge von 0 bis 2 Gew.-% in der Trockenmasse der folienförmigen Zubereitung enthalten.

In einer besonders bevorzugten Ausführungsform weist der Wafer Coffein auf, das der polymerhaltigen Masse bei der Herstellung der Wafer zugesetzt wurde. Coffein wird ohnehin mit dem Genuß von Cola assoziiert und kann bei den erfindungsgemäßen Wafern zu einer Verbesserung des geschmacklichen Gesamteindrucks führen. Vorzugsweise ist Coffein in einer Menge von 0 bis 2 Gew.-% in der Trockenmasse der folienförmigen Zubereitung enthalten.

Zusätzlich können bis zu 20 Gew.-% eines oder mehrerer Füllstoffe, z. B. als Verdicker, Zerfallsbeschleuniger oder Stabilisator, und bis zu 5 Gew.-% eines oder mehrerer Konservierungsstoffe (z.B. Antioxidantien, Kristallisationsinhibitoren und Konservierungsmittel gegen mikrobiellen Verderb), jeweils bezogen auf die Trockenmasse der folienförmigen Zubereitung, in den erfindungsgemäßen Wafern enthalten sein.

Die bevorzugten Ausführungsformen der erfindungsgemäßen Wafer setzen sich aus den folgenden Inhaltsstoffen in den jeweils angegebenen Mengenbereichen zusammen:

| Trockenanteil (Gew.-%) | Bestandteil |
|---|---|
| 55 - 75 | filmbildendes Polymer(gemisch) |
| 5 - 20 | Cola-Aroma |
| 0 - 18 | Weichmacher |
| 0 - 19 | Süßstoff |
| 0 - 7 | Emulgator |
| 0 - 2 | Farbstoff |
| 0 - 5 | Säuerungsmittel |
| 0 - 3 | weitere Aromen |
| 0 - 5 | Konservierungsmittel |
| 0 - 20 | Füllstoff(e) |

Mit Hilfe von Versuchen wurden Zusammensetzungen mit besonders bevorzugten Eigenschaften ermittelt, bei denen sich die folgenden Bestandteile in den angegebenen Mengenbereichen bewegten:

| Trockenanteil (Gew.-%) | Bestandteil | Hauptfunktion |
|---|---|---|
| 55 - 65 | hydroxypropylierte Tapiokastärke | filmbildendes Polymer |
| 0 - 10 | teilhydrolysierter Polyvinylalkohol | filmbildendes Polymer |
| 5 - 20 | Cola-Aroma | Aroma |
| 5 - 18 | Glycerin | Weichmacher |
| 0 - 18 | Sorbitol-Sirup | Süßstoff |
| 0 - 1 | Sucralose | Süßstoff |
| 0 - 1 | Mono- und Diglyceride v. Fettsäuren | Emulgator |
| 0 - 3 | Polyethoxylierte Sorbitanester | Emulgator |
| 0 - 3 | Macrogolglycerolhydroxystearat | Emulgator |
| 0,2 - 2 | Zuckerkulör | Farbstoff |
| 2 - 5 | Zitronensäure | Säuerungsmittel |
| 0 - 1 | Limetten-Aroma | Aroma |
| 0,1 - 2 | Coffein | Aroma |

Als geschmacklich ganz besonders ansprechende Variante hat sich die in Beispiel 3 angegebene Zusammensetzung herausgestellt.

### Beispiel 1

Es wurde eine polymerhaltige Masse, bestehend aus 25 Gew.-% Polyvinylpyrrolidon und 75 Gew.-% einer im Einzelhandel als Erfrischungsgetränk erhältlichen Cola hergestellt, auf einer Unterlage ausgestrichen, durch Trocknung in eine Folie überführt und durch Vereinzelung in bis zu 10 cm² große Wafer konfektioniert.

Diese Folie schmeckte jedoch nicht nach Cola.

### Beispiel 2

Es wurde eine Mischung mit der folgenden Zusammensetzung hergestellt:

| Trockenanteil [Gew.-%] | Bestandteil |
|---|---|
| 10,95 | PVA |
| 0,90 | Zitronensäure |
| 0,12 | Tween^{®} 80 |
| 0,03 | Physcool^{®} |
| 57,14 | Cola-Sirup |
| 30,86 | Wasser |

Diese Mischung wurde auf einer Unterlage ausgestrichen, durch Trocknung in eine Folie überführt und durch Vereinzelung in bis zu 10 cm² große Wafer konfektioniert.

Die Wafer schmeckten zwar deutlich nach Cola, hatten aber einen unangenehm süßen Geschmack.

### Beispiel 3

Es wurde eine Mischung mit der folgenden Zusammensetzung hergestellt:

| Anteil (Gew.-%) | Bestandteil |
|---|---|
| 28,4 | C*AraSet (hydroxypropylierte Tapiokastärke) |
| 2,5 | Mowiol, 5 - 6 mPas (teilhydrolysierter Polyvinylalkohol) |
| 5,5 | Glycerin |
| 5,0 | Sorbidex |
| 0,2 | Sucralose |
| 0,3 | Atmos® 300 (Mono- und Diglyceride von Speisefettsäuren) |
| 0,8 | Tween® 80 (Polyethylensorbitanmonooleat) |
| 0,8 | Cremophor® RH 40 (Macrogolglycerolhydroxystearat) |
| 1,7 | E 150 |
| 2,0 | Zitronensäure |
| 0,3 | Limetten-Aroma |
| 0,2 | Coffein |
| 5,0 | Cola-Aroma |
| 47,3 | Wasser |

Diese Mischung wurde auf einer Unterlage ausgestrichen, durch Trocknung in eine Folie überführt und durch Vereinzelung in bis zu 10 cm² große Wafer konfektioniert.

Die resultierenden Wafer lösten sich schnell und rückstandsfrei im Mund auf, führten nicht zu einem unangenehm klebrigen Mundgefühl und zeigten einen erfrischenden Cola-Geschmack.

## Patentansprüche

1. Eßbare, wasserlösliche, folienförmige Zubereitung, welche Cola-Aroma enthält, **dadurch gekennzeichnet, daß** sie sich bei Kontakt mit Feuchtigkeit schnell und rückstandsfrei auflöst, und hydroxypropylierte Tapiokastärke, oder hydroxypropylierte Tapiokastärke in Kombination mit weiteren filmbildenden Polymeren, enthält.

2. Zubereitung nach Anspruch 1, **dadurch gekennzeichnet, daß** die weiteren filmbildenden Polymere aus der aus Cellulosederivaten, teilhydrolysierte Polyvinylalkoholen, Polyvinylpyrrolidon, Gelatine, Alginaten und Polyethylenglykolen bestehenden Gruppe ausgewählt sind.

3. Zubereitung nach Anspruch 1, **gekennzeichnet durch** die folgende Zusammensetzung:
| | |
|---|---|
| 55 - 75 Gew.-% | hydroxypropylierte Tapiokastärke |
| 5 - 20 Gew.-% | Cola-Aroma |
| 0 - 18 Gew.-%. | Weichmacher, |
| 0 - 19 Gew.-% | süßstoff |
| 0 - 7 Gew.-% | Emulgator |
| 0 - 2 Gew.-% | Farbstoff |
| 0 - 5 Gew.-% | Säuerungsmittel |
| 0 - 3 Gew.-% | Weiteres Aroma |
| 0 - 5 Gew.-% | Konservierungsmittel |
| 0 - 20 Gew.-% | Füllstoff(e) |

4. Zubereitung nach Anspruch 1, **dadurch gekennzeichnet, daß** das filmbildende Polymergemisch aus
a) 55 - 65 Gew.-% hydroxypropylierter Tapiokastärke und
b) 0,01 bis 10 Gew.-% eines oder mehrerer weiterer filmbildender Polymere besteht, das/die aus der Gruppe ausgewählt ist/sind, die Natriumcarboxymethylcellulose, Hydroxypropylmethylcellulose, Hydroxypropylcellulose, teilhydrolysierte Polyvinylalkohole, Polyvinylpyrrolidon, Gelatine, Alginate, Polyethylenglykole, wasserlösliche Stärkeanteile und andere wasserlöslichen Stärkederivate als das unter a) genannte hydroypropylierte stärkederivat umfaßt.

5. Zubereitung nach einem der Ansprüche 1 bis 4, **dadurch gekennzeichnet, daß** sie hydroxypropylierte Tapiokastärke und teilhydrolysierten Polyvinylalkohol enthält.

6. Zubereitung nach einem der vorangehenden Ansprüche, **dadurch gekennzeichnet, daß** sie mindestens einen Weichmacher enthält, der aus der Gruppe ausgewählt ist, die Ethylenglycole, Polyethylenglycole, Dibutylsebacat, Diethylphthalat, diacetylierte Monoglyceride, Triacetin, Tributylcitrat, Triethylcitrat, Acetyltributylcitrat, Acetyltriethylcitrat, Benzoylbenzoat, Propylenglycol, Ricinusöl, Saccharose, Isomalt, Mannitol, Stärkezucker und Dexpanthenol umfaßt.

7. Zubereitung nach einem der vorangehenden Ansprüche, **dadurch gekennzeichnet, daß** sie mindestens einen Emulgator enthält, der aus der Gruppe ausgewählt ist, die die Mono-und Diglyceride von Speisefettsäuren, Polyethylenglykolether, Sorbitanfettsäureester, Polysorbate, Pectine, Lecithin und Macrogolglycerolhydroxystearat umfaßt.

8. Zubereitung nach einem der vorangehenden Ansprüche, **dadurch gekennzeichnet, daß** sie mindestens einen süßstoff enthält, der aus der Gruppe ausgewählt ist/sind, die Acesulfam, Aspartam, Cyclamat, Saccharin, Sorbitol, Sucralose (Trichlorsucrose), Thaumatin, Neohesperidin DC und Mischungen dieser Süßstoff umfaßt.

9. Zubereitung nach einem der vorangehenden Ansprüche, **dadurch gekennzeichnet, daß** sie ein Säuerungsmittel enthält, das aus der Gruppe ausgewählt, ist/sind, die Weinsäure, Ascorbinsäure, Äpfelsäure, Phosphorsäure, Milchsäure und Zitronensäure umfaßt.

10. Zubereitung nach einem der vorangehenden Ansprüche, **gekennzeichnet durch** die folgende Zusammensetzung, bezogen auf die Trockenmasse der Zubereitung:
| | |
|---|---|
| 55 - 65 Gew.-% | hydroxypropylierte Tapiokastärke) |
| 0 - 10 Gew.-% | teilhydrolysierter Polyvinylalkohol |
| 5 - 18 Gew.-% | Glycerin |
| 0 - 18 Gew. -% | sorbitol-sirup |
| 0,2 - 1 Gew.-% | Sucralose |
| 0 - 1 Gew.-% | Mono- und Diglyceride von Fettsäuren |
| 0 - 3 Gew.-% | Polyoxyethylensorbitanmonooleat |
| 0 - 3 Gew.-% | Macrogolglycerolhydroxystearat |
| 0,2 - 2 Gew.-% | Zuckerkulör |
| 2 - 5 Gew.-% | Zitronensäure |
| 0 - 1 Gew.-% | Limetten-Aroma |
| 0,1 - 2 Gew.-% | Coffein |
| 5 - 20 Gew.-% | Cola-Aroma |

11. Zubereitung nach Anspruch 9, **gekennzeichnet durch** die folgende Zusammensetzung, bezogen auf die Trockenmasse der Zubereitung:
| | |
|---|---|
| 56,9 Gew.-% | hydroxypropylierte Tapiokastärke |
| 5,0 Gew.-% | teilhydrolisierter Polyvinylalkohol |
| 11,0 Gew.-% | Glycerin |
| 7.0 Gew.-% | Sorbitol-Sirup |
| 0,5 Gew.-% | Sucralose |
| 0,5 Gew.-% | Mono- und Diglyceride von Speisefettsäuren |
| 1,5 Gew.-% | Polyoxyethylensorbitanmonooleat |
| 1,5 Gew.-% | Macrogolglycerolhydroxystearat |
| 1,2 Gew.-% | Zuckerkulär |
| 4,0 Gew.-% | Zitronensäure |
| 0, 5 Gew.-% | Limetten-Aroma |
| 0,4 Gew.-% | Coffein |
| 10,0 Gew.-% | cola-Aroma |

12. Verwendung von Cola-Aroma in Kombination mit hydroxypropylierter Tapiokastärke zur Herstellung einer eßbaren, wasserlöslichen folienförmigen Zubereitung, die sich bei Kontakt mit Feuchtigkeit schnell und rückstandsfrei auflöst.

## Claims

1. An edible, water-soluble, film-shaped preparation containing cola flavouring, wherein said preparation dissolves quickly upon contact with moisture and does not leave a residue, and contains hydroxypropylated tapioca starch, or hydroxypropylated tapioca starch in combination with further film-forming polymers.

2. The preparation according to claim 1, wherein said further film-forming polymers are selected from the group consisting of cellulose derivatives, partially hydrolysed polyvinyl alcohols, polyvinyl pyrrolidone, gelatine, alginates and polyethylene glycols.

3. The preparation according to claim 1, wherein said preparation comprises the following composition:
| | |
|---|---|
| 55-75%-wt. | hydroxypropylated tapioca starch |
| 5-20%-wt. | cola flavouring |
| 0-18%-wt. | plasticiser |
| 0-19%-wt. | sweetener |
| 0-7%-wt. | emulsifier |
| 0-2%-wt. | colouring |
| 0-5%-wt. | acidifier |
| 0-3%-wt. | further flavouring |
| 0-5%-wt. | preservative |
| 0-20%-wt. | filler(s). |

4. The preparation according to claim 1, wherein the film-forming polymer mixture comprises
a) 55 to 65%-wt. of a hydroxypropylated tapioca starch, and
b) 0.01 to 10%-wt. of one or more further film-forming polymers, which is/are selected from the group consisting of sodium carboxymethyl cellulose, hydroxypropylmethyl cellulose, hydroxypropyl cellulose, partially hydrolyzed polyvinyl alcohols, polyvinyl pyrrolidone, gelatine, alginates, polyethylene glycols, water-soluble starch portions, and water-soluble starch derivatives other than the hydroxypropylated starch derivative mentioned under a).

5. The preparation according to one of claims 1 to 4, which comprises hydroxypropylated tapioca starch and partially hydrolysed polyvinyl alcohol.

6. The preparation according to one of the preceding claims, which comprises at least one plasticizer selected from the group consisting of ethylene glycols, polyethylene glycols, dibutyl sebacate, diethyl phthalate, diacetylated monoglycerides, triacetin, tributyl citrate, triethyl citrate, acetyl tributyl citrate, acetyl triethyl citrate, benzoyl benzoate, propylene glycol, castor oil, saccharose, isomalt, mannitol, starch sugars and dexpanthenol.

7. The preparation according to one of the preceding claims, which comprises at least one emulsifier selected from the group consisting of the mono-and diglycerides of edible fatty acids, polyethylene glycol ethers, sorbitan fatty acid esters, polysorbates, pectins, lecithin and macrogol glycerol hydroxystearate.

8. The preparation according to one of the preceding claims, which comprises at least one sweetener selected from the group consisting of acesulfame, aspartame, cyclamate, saccharin, sorbitol, sucralose (trichlorosucrose), thaumatin, neohesperidin DC, and mixtures of these sweeteners.

9. The preparation according to one of the preceding claims, which comprises an acidifier selected from the group consisting of tartaric acid, ascorbic acid, malic acid, phosphoric acid, lactic acid and citric acid.

10. The preparation according to one of the preceding claims, wherein said preparation comprises the following composition, relative to the dry matter of the preparation:
| | |
|---|---|
| 55-65%-wt. | hydroxypropylated tapioca starch |
| 0-10%-wt. | partially hydrolysed polyvinyl alcohol |
| 5-18%-wt. | glycerine |
| 0-18%-wt. | sorbitol syrup |
| 0.2-1%-wt. | sucralose |
| 0-1%-wt. | mono- and diglycerides of fatty acids |
| 0-3%-wt. | polyoxyethylene sorbitan monooleate |
| 0-3%-wt. | macrogol glycerol hydroxystearate |
| 0.2-2%-wt. | caramel colouring |
| 2-5%-wt. | citric acid |
| 0-1%-wt. | lime flavouring |
| 0.1-2%-wt. | caffeine |
| 5-20%-wt. | cola flavouring. |

11. The preparation according to claim 9, wherein said preparation comprises the following composition, relative to the dry matter of the preparation:
| | |
|---|---|
| 56.9%-wt. | hydroxypropylated tapioca starch |
| 5.0%-wt. | partially hydrolysed polyvinyl alcohol |
| 11.0%-wt. | glycerine |
| 7.0%-wt. | sorbitol syrup |
| 0.5%-wt. | sucralose |
| 0.5%-wt. | mono- and diglycerides of edible fatty acids |
| 1.5%-wt. | polyoxyethylene sorbitan monooleate |
| 1.5%-wt. | macrogol glycerol hydroxystearate |
| 1.2%-wt. | caramel colouring |
| 4.0%-wt. | citric acid |
| 0.5%-wt. | lime flavouring |
| 0.4%-wt. | caffeine |
| 10.0%-wt. | cola flavouring. |

12. Use of cola flavouring, in combination with hydroxypropylated tapioca starch, for producing an edible, water-soluble film-shaped preparation which dissolves quickly upon contact with moisture and does not leave a residue.

## Revendications

1. Préparation pelliculaire, soluble dans l'eau, comestible, qui contient de l'arôme cola, **caractérisée en ce qu'**au contact de l'humidité elle se dissout rapidement et sans résidu, et contient de l'amidon de tapioca hydroxypropylé, ou de l'amidon de tapioca hydroxypropylé en association avec d'autres polymères filmogènes.

2. Préparation selon la revendication 1, **caractérisée en ce que** les autres polymères filmogènes sont choisis dans le groupe constitué par des dérivés de cellulose, des poly(alcool vinylique)s partiellement hydrolysés, la polyvinylpyrrolidone, la gélatine, les alginates et les polyéthylèneglycols.

3. Préparation selon la revendication 1, **caractérisée par** la composition suivante :
| | |
|---|---|
| 55 - 75 % | en poids d'amidon de tapioca hydroxypropylé |
| 5 - 20 % | en poids d'arôme cola |
| 0 - 18 % | en poids de plastifiant |
| 0 - 19 % | en poids d'édulcorant |
| 0 - 7 % | en poids d'émulsifiant |
| 0 - 2 % | en poids de colorant |
| 0 - 5 % | en poids d'acidifiant |
| 0 - 3 % | en poids d'autre arôme |
| 0 - 5 % | en poids de conservateur |
| 0 - 20 % | en poids d'excipient(s). |

4. Préparation selon la revendication 1, **caractérisée en ce que** le mélange de polymères filmogènes est constitué de
a) 55 - 65 % en poids d'amidon de tapioca hydroxypropylé et
b) 0,01 à 10 % en poids d'un ou plusieurs autres polymères filmogènes, qui est/sont choisi(s) dans le groupe qui comprend la carboxyméthylcellulose sodique, l'hydroxypropylméthylcellulose, l'hydroxypropylcellulose, des poly(alcool vinylique)s partiellement hydrolysés, la polyvinylpyrrolidone, la gélatine, des alginates, des polyéthylèneglycols, des fractions d'amidon hydrosolubles et des dérivés d'amidon hydrosolubles autres que le dérivé d'amidon hydroxypropylé nommé en a).

5. Préparation selon l'une quelconque des revendications 1 à 4, **caractérisée en ce qu'**elle contient de l'amidon de tapioca hydroxypropylé et du poly(alcool vinylique) partiellement hydrolysé.

6. Préparation selon l'une quelconque des revendications précédentes, **caractérisée en ce qu'**elle contient au moins un plastifiant, qui est choisi dans le groupe qui comprend des éthylèneglycols, des polyéthylèneglycols, le sébaçate de dibutyle, le phtalate de diéthyle, des monoglycérides diacétylés, la triacétine, le citrate de tributyle, le citrate de triéthyle, le citrate d'acétyltributyle, le citrate d'acétyltriéthyle, le benzoate de benzoyle, le propylèneglycol, l'huile de ricin, le saccharose, l'isomalt, le mannitol, le sucre d'amidon et le dexpanthénol.

7. Préparation selon l'une quelconque des revendications précédentes, **caractérisée en ce qu'**elle contient au moins un émulsifiant, qui est choisi dans le groupe qui comprend les mono- et diglycérides d'acides gras alimentaires, les éthers de polyéthylèneglycol, les esters d'acides gras et de sorbitanne, les polysorbates, les pectines, la lécithine et l'hydroxystéarate de macrogol-glycérol.

8. Préparation selon l'une quelconque des revendications précédentes, **caractérisée en ce qu'**elle contient au moins un édulcorant, qui est/sont choisi(s) dans le groupe qui comprend l'acésulfame, l'aspartame, le cyclamate, la saccharine, le sorbitol, le sucralose (trichlorosaccharose), la thaumatine, la néohespéridine DC et des mélanges de ces édulcorants.

9. Préparation selon l'une quelconque des revendications précédentes, **caractérisée en ce qu'**elle contient un acidifiant, qui est choisi dans le groupe qui comprend l'acide d-tartrique, l'acide ascorbique, l'acide malique, l'acide phosphorique, l'acide lactique et l'acide citrique.

10. Préparation selon l'une quelconque des revendications précédentes, **caractérisée par** la composition suivante, par rapport à la matière sèche de la préparation :
| | |
|---|---|
| 55 - 65 % en poids | d'amidon de tapioca hydroxypropylé |
| 0 - 10 % en poids | de poly(alcool vinylique) partiellement hydrolysé |
| 5 - 18 % en poids | de glycérol |
| 0 - 18 % en poids | de sirop de sorbitol |
| 0,2 - 1 % en poids | de sucralose |
| 0 - 1 % en poids | de mono- et diglycérides d'acides gras |
| 0 - 3 % en poids | de mono-oléate polyoxyéthylénique de sorbitanne |
| 0 - 3 % en poids | d'hydroxystéarate de macrogol-glycérol |
| 0,2 - 2 % en poids | de colorant caramel |
| 2 - 5 % | en poids d'acide citrique |
| 0 - 1 % | en poids d'arôme citron vert |
| 0,1 - 2 % | en poids de caféine |
| 5 - 20 % | en poids d'arôme cola. |

11. Préparation selon la revendication 9, **caractérisée par** la composition suivante, par rapport à la matière sèche de la préparation :
| | |
|---|---|
| 56,9 % | en poids d'amidon de tapioca hydroxypropylé |
| 5,0 % | en poids de poly(alcool vinylique) partiellement hydrolysé |
| 11,0 % | en poids de glycérol |
| 7,0 % | en poids de sirop de sorbitol |
| 0,5 % | en poids de sucralose |
| 0,5 % | en poids de mono- et diglycérides d'acides gras alimentaires |
| 1,5 % | en poids de mono-oléate polyoxyéthylénique de sorbitanne |
| 1,5 % | en poids d'hydroxystéarate de macrogol-glycérol |
| 1,2 % | en poids de colorant caramel |
| 4,0 % | en poids d'acide citrique |
| 0,5 % | en poids d'arôme citron vert |
| 0,4 % | en poids de caféine |
| 10,0 % | en poids d'arôme cola. |

12. Utilisation d'arôme cola en association avec de l'amidon de tapioca hydroxypropylé pour la fabrication d'une préparation pelliculaire, soluble dans l'eau, comestible, qui au contact de l'humidité se dissout rapidement et sans résidu.
